Europäisches Patentamt

European Patent Office

Office européen des brevets

(11) Publication number: **0 564 804 A1**

# EUROPEAN PATENT APPLICATION

(21) Application number: 93103174.4

(22) Date of filing: 27.02.93

(51) Int. Cl.⁵: **A23L 1/305**, A23L 1/304, A61K 9/00, A61K 9/08

(30) Priority: 10.04.92 US 866833

(43) Date of publication of application:
13.10.93 Bulletin 93/41

(84) Designated Contracting States:
AT BE CH DE DK ES FR GB GR IE IT LI LU NL PT SE

(71) Applicant: CLINTEC NUTRITION COMPANY
Three Parkway North,
Suite 500
Deerfield, Illinois 60015(US)

(72) Inventor: Trimbo, Susan L.
737 Ridge Avenue 3F
Evanston, IL 60202(US)
Inventor: Twyman, Diana
40 East Oak
Chicago, IL 60611(US)

(74) Representative: Ledzion, Andrzej et al
Avenue Nestlé 55
CH-1800 Vevey (CH)

(54) Improved high protein liquid nutrition for patients with elevated wound healing requirements.

(57) The present invention provides a high protein liquid nutrition formula for patients with elevated wound healing requirements and a method for treating such patients. The high protein formula comprises per 1,000 Kcal: a protein source; a fat source; a carbohydrate source; a zinc source providing at least approximately 150% of the USRDA of zinc; a Vitamin C source providing at least approximately 500% of the USRDA of Vitamin C; a selenium source providing over approximately 100 mcg of selenium; a Vitamin A source providing at least approximately 145% of the USRDA of Vitamin A including a beta-carotene source providing at least approximately 2.0 mg of beta-carotene; and a source of thiamine providing at least approximately 200% of the USRDA of thiamine.

EP 0 564 804 A1

## BACKGROUND OF THE INVENTION

The present invention relates generally to liquid nutritional products. More specifically, the present invention relates to high protein liquid nutritional products.

A number of high protein liquid nutritional products exist for use in meeting certain requirements of patients. For example, Replete® high protein liquid nutrition and Replete® with fiber complete high protein liquid nutrition, both available from Clintec Nutrition Company, Deerfield, Illinois, provide ready-to-use formulas for the complete, or supplemental, tube feeding of a patient. These products provide nutritional formulas that can support the healing or repletion needs of a patient.

Other formulations that are available include Traumacal® by Mead Johnson that is a high protein formula allegedly designed to meet the special metabolic needs of a trauma patient. Impact® from Sandoz also allegedly provides a nutritional product for trauma patients. Another available nutritional product is Jevity from Ross.

Clinicians have noted that extremely malnourished patients often have delayed wound healing. Wound healing is a complex process for growth and regeneration. Wound healing is affected to a great extent by metabolic and nutritional factors. Indeed, even non-overt malnutrition can have physiological effects on wound healing.

One of the difficulties in insuring that a patient is provided with sufficient nutrition during increased wound healing requirements is that certain key metabolic nutritional abnormalities may occur acutely and may not be accompanied by a specific, readily recognizable symptom or physical sign. They would only be recognizable by biochemical, physiological, and immunological measurements. However, wound healing may be affected.

In certain circumstances and for certain types of patients, presently available, high nutritional products may not meet all of the necessary nutritional and metabolic needs of the patient. This is true even with respect to formulations specifically designed for trauma.

## SUMMARY OF THE INVENTION

The present invention provides a product that is specifically directed to meet the special metabolic needs of patients with increased wound healing requirements. The present invention is specifically directed to patients having elevated wound healing requirements that may be due to the following conditions: trauma; burns; pressure ulcers; post-surgical wound care; cancer; and repletion of lean body mass losses of greater than 15%.

To this end, the present invention provides a high protein liquid nutrition for patients with elevated wound healing requirements comprising per 1,000 Kcal: a protein source; a fat source; a carbohydrate source; a zinc source providing at least approximately 150% of the USRDA of zinc; a Vitamin C source providing at least approximately 500% of the USRDA of Vitamin C; a selenium source providing at least approximately 100 mcg of selenium; a Vitamin A source providing at least approximately 145% of the USRDA of Vitamin A, the vitamin A source includes a beta-carotene source providing at least approximately 1.5 mg of beta-carotene; and a source of thiamine providing at least approximately 200% of the USRDA of thiamine.

In an embodiment, the high protein liquid nutrition composition includes a source of fiber.

In an embodiment of the high protein liquid nutrition composition, the fat source includes approximately 0% to about 30% of the total caloric fat content as MCTs. Preferably, the composition includes 25% of the caloric content of the fat source as MCTs.

Further, with respect to the fat source, in an embodiment, preferably, the ratio of n-6 to n-3 fatty acids is 2:1 to 10:1.

In an embodiment, the liquid nutrition composition includes at least approximately 100% of the USRDA of calcium and phosphorus.

In an embodiment, the liquid nutrition composition includes at least approximately 140 mcg of chromium and approximately 220 mcg of molybdenum per 1,000 Kcal.

In an embodiment, the elevated wound healing requirements is required in the patient because of: trauma; cancer; burns; pressure ulcers; surgery; or loss of lean body mass greater than 15%.

An advantage of the present invention is that it provides high protein to meet elevated wound healing requirements.

Furthermore, an advantage of the present invention is that it provides 100% or more of the USRDA in 1,000 calories.

A further advantage of the present invention is that it provides high levels of Vitamin C and zinc to support wound healing and provide for possible increased requirements.

Still further, an advantage of the present invention is that it is rich in beta-carotene to meet necessary requirements, prevent deficiencies, and meet possible requirements in long-term, tube fed patients.

Additionally, an advantage of the present invention is that the nutrition composition is rich in n-3 fatty acids which may reduce immune suppression associated with high n-6 fatty acid concentrations.

Further, an advantage of the present invention is that it provides increased levels of thiamine and other B vitamins to support elevated requirements and treat borderline deficiencies that may interfere with wound healing.

Moreover, an advantage of the present invention is that it contains taurine, carnitine, and ultra trace minerals to prevent deficiencies in long-term, tube fed patients and support elevated healing requirements.

Additional features and advantages of the present invention are described in, and will be apparent from, the detailed description of the presently preferred embodiments.

## DETAILED DESCRIPTION OF THE PRESENTLY PREFERRED EMBODIMENTS

The present invention provides nutritional product for meeting the special metabolic needs of patients with elevated healing requirements.

The present invention comprises at least approximately 18% of the total calories as protein. In a preferred embodiment, approximately 25% of the calories are present as a protein. Preferably, the protein source is casein. However, other protein sources can be used including whey, total milk protein, and the like.

Elevated protein requirements have been identified in patient populations such as pressure ulcer, serious wounds, trauma, or where wasting has occurred. Inherent to the metabolic requirements of these conditions is an increased loss in nitrogen, increased requirement for protein, or both.

In addition to requirements for high protein as a percentage of total calories, increased nitrogen losses and elevated requirements of certain vitamins, minerals, and trace elements may occur. Guidelines for vitamins, minerals, and trace elements in wound healing patients do not exist due to the difficulty of performing well controlled clinical research in these populations.

Pursuant to the present invention, the formula of the present invention includes a high level of zinc. Preferably, at least approximately 150% of the USRDA of zinc is provided in the composition per 1,000 Kcal. In a preferred embodiment, 24-27 milligrams per 1,000 calories of zinc are provided (160 to 208% of the USRDA). The increased zinc makes up for zinc losses and provides increased zinc for tissue repair in a patient having increased healing requirements.

Pursuant to the present invention, at least approximately 500% of the USRDA of Vitamin C is provided per 1,000 Kcal. In a preferred embodiment, 340 milligrams per 1,000 calories of Vitamin C is provided (540% of USRDA). Vitamin C is believed to accelerated the wound healing and granulation in patients with severe wound healing requirements. Vitamin C will support increased requirements/losses after surgery.

The present invention also provides increased amounts of selenium. Selenium deficiencies may develop in patients having elevated wound healing requirements despite selenium intakes within the range of normal healthy adults (106-146 micrograms per day). Pursuant to the present invention, at least approximately 100 micrograms of selenium are provided in 1,000 calories of formula per day. Although a USRDA requirement for selenium does not exist, it is believed by some to be around 50 mcg a day for a normal healthy adult.

Although many commercially available enteral formulations provide more than adequate supplies of Vitamin A for healthy individuals, patients suffering from a variety of illness may have higher requirements. Further, Vitamin A may be mobilized from liver stores more poorly due to lower synthesis of serum retinol binding protein. Indeed, retinol binding protein levels decreases may be associated with protein-energy malnutrition and zinc deficiency.

Vitamin A can stimulate the synthesis of connective tissue. Conversely, Vitamin A deficiency leads to impaired collagen synthesis. Additionally, Vitamin A has been shown to overcome the inhibitory effects of cortisone on the rate of gain in tensile strength in early stages of healing. In this capacity, Vitamin A maintains and restores the inflammatory stimulus required to generate healing factors for wound repair. The present invention provides at least 145% of the USRDA per 1,000 Kcal of Vitamin A.

Nearly all, if not all, of the major currently commercially available enteral formulas contain far below the amounts of carotenoids (beta-carotene) found in the usual diets of normal healthy people. In fact, patients on liquid formula diets as their sole source of nutrition for one week or more have been found to have plasma concentrations of carotenoids of only 8 to 18 percent as compared to controls consuming a free choice diet. See, Bowen et al, Hypocarotenemia in Patients Fed Enterally with Commercial Liquid Diets, JPEN, 1988; 12(5):484-489. Those on enteral formulas for more than 3 weeks have negligible concentrations of any common serum carotinoids.

To meet these requirements, the present invention provides at least 2.0 milligrams of beta-carotene per 1,000 calories. Preferably, 2 milligrams of beta-carotene per 1,000 calories are provided. This prevents deficiencies and provides for possible increased requirements in the wound healing patient. The beta-carotene levels allows plasma concentrations to be increased to near normal optimal levels of 500 mcg per liter.

The present invention also provides increased thiamine to meet requirements and treat marginal thiamine deficiencies found in most geriatric patients. Further, the increased thiamine is desirable because water soluble vitamin requirements may increase during healing. In an embodiment, at least approximately 200% of the USRDA for thiamine is provided in 1,000 calories. In a presently preferred embodiment, approximately 200% of the USRDA for thiamine is provided per 1,000 calories of composition.

The present invention also provides at least approximately 100% of the USRDA, at 1,000 Kcal, of necessary trace elements molybdenum and chromium. In a preferred embodiment, approximately 140 mcg of chromium is provided and approximately 200 mcg of molybdenum is provided per 1,000 Kcal.

Additionally, at least approximately 150% of USRDA at 1,000 Kcal of Vitamin E is provided. The fat soluble vitamins have been associated with improved healing.

The present invention provides a fat source. In a preferred embodiment, the fat source includes, as a percent of the caloric distribution 0 to 30% MCTs. In a presently preferred embodiment, approximately 25% of the calories of the fat source are provided as MCTs.

Additionally, the fat source includes preferably a ratio of n-6 to n-3 fatty acids of approximately 2:1 to about 10:1. Preferably, the ratio of n-6 to n-3 fatty acids is approximately 2.5 to 1. The ratio is preferably achieved using canola oil. However, other oils such as, for example, soy bean oil, olive oil, corn oil, marine oil, or mixtures thereof, can be used.

The present invention can be used to treat elevated wound healing requirements. As used herein, elevated wound healing requirements can be caused by the following conditions: trauma; burns; pressure ulcers; post surgical wound care; cancer; and losses of lean body masses of greater than 20%.

Typically, on average, approximately 2,000 Kcal of composition will be given per day to a patient with elevated wound healing requirements. Of course, some patients with very high requirements may require substantially more composition and some patients with lower requirements, and/or weights, may require less composition.

By way of example, and not limitation, examples of the present invention will now be given:

| Nutrients | Unit (Per liter) | Example of Composition of Present Invention | Example of Composition with Fiber | % USRDA (Per liter) |
|---|---|---|---|---|
| Protein | g | 62.5 | 62.5 | 140 |
| Fat | g | 34.0 | 34.0 | |
| MCT oil | g | 8.4 (25%) | 8.4 (25%) | |
| Canola oil | g | 23.6 (69%) | 23.6 (69%) | |
| Lecithin | g | 2.0 (6%) | 2.0 (6%) | |
| CHO | g | 113 | 113 | |
| Dietary Fiber | g | 0 | 14 | |
| Energy value | Kcal | 1000 | 1000 | |
| Water | g | 845 | 836 | |
| Vitamin A | IU | 7333* | 7333* | 147 |
| *(Includes 2 mg or 3333 IU from beta-carotene) | | | | |
| Vitamin D | IU | 400 | 400 | 100 |
| Vitamin E | IU | 60 | 60 | 200 |
| Vitamin K | mcg | 80 | 80 | * |
| Vitamin C | mg | 340 | 340 | 567 |
| Thiamine (B1) | mg | 3 | 3 | 200 |
| Riboflavin (B2) | mg | 2.4 | 2.4 | 141 |
| Niacin | mg | 28 | 28 | 140 |
| Vitamin B6 | mg | 4 | 4 | 200 |
| Folic Acid | mcg | 540 | 540 | 135 |
| Pantothenic Acid | mg | 14 | 14 | 140 |
| Vitamin B12 | mcg | 8 | 8 | 133 |
| Biotin | mcg | 400 | 400 | 133 |
| Choline | mg | 450 | 450 | * |
| Taurine | mg | 100 | 100 | * |
| L-Carnitine | mg | 100 | 100 | * |
| Calcium | mg | 1000 | 1000 | 100 |
| Phosphorus | mg | 1000 | 1000 | 100 |
| Magnesium | mg | 400 | 400 | 100 |
| Iron | mg | 18 | 18 | 100 |
| Zinc | mg | 24 | 24 | 160 |
| Copper | mg | 2 | 2 | 100 |
| Manganese | mg | 4 | 4 | * |
| Iodine | mcg | 160 | 160 | 107 |
| Sodium | mg | 500 | 500 | * |
| Potassium | mg | 1560 | 1560 | * |
| Chloride | mg | 1000 | 1000 | * |
| Chromium | mcg | 140 | 140 | * |
| Molybdenum | mcg | 220 | 220 | * |
| Selenium | mcg | 100 | 100 | * |
| Osmolality | mOsm/Kg | 290 | 300 | |

Dietary fiber can be present as soy polysaccharide.

It should be understood that various changes and modifications to the presently preferred embodiments described herein will be apparent to those skilled in the art. Such changes and modifications can be made without departing from the spirit and scope of the present invention and without diminishing its attendant advantages. It is therefore intended that such changes and modifications be covered by the appended claims.

**Claims**

1. A high protein liquid nutrition formula for patients with elevated wound healing requirements comprising:
   a protein source;
   a fat source;
   a carbohydrate source;
   a Vitamin C source providing at least 500% of the U.S. RDA of Vitamin C per 1,000 Kcal of formula; and
   a Vitamin A source providing at least 145% of the U.S. RDA of Vitamin A per 1,000 Kcal of formula.

2. A high protein liquid nutrition formula for patients with elevated wound healing requirements comprising:
   a protein source;
   a carbohydrate source;
   a fat source;
   at least 150% of the U.S. RDA of zinc per 1,000 Kcal of formula;
   at least 500% of the U.S. RDA of Vitamin C per 1,000 Kcal of formula;
   at least 100 mcg of selenium per 1,000 Kcal of formula;
   at least 145% of the U.S. RDA of Vitamin A per 1,000 Kcal of formula including at least 2 mg of beta-carotene per 1,000 Kcal of formula; and
   at least 200% of the U.S. RDA of thiamine per 1,000 Kcal of formula.

3. A high protein liquid nutrition formula for patients with elevated wound healing requirements comprising:
   a protein source that provides at least 18% of the total calories of the formula;
   a carbohydrate source;
   a fat source including medium chain triglycerides;
   at least 150% of the U.S. RDA of zinc per 1,000 Kcal of formula;
   at least 500% of the U.S. RDA of Vitamin C per 1,000 Kcal of formula;
   at least 100 mcg of selenium per 1,000 Kcal of formula;
   at least 145% of the U.S. RDA of Vitamin A per 1,000 Kcal of formula including at least 2 mg of beta-carotene per 1,000 Kcal of formula;
   at least 200% of the U.S. RDA of thiamine per 1,000 Kcal of formula; and
   a source of dietary fiber.

4. The high protein liquid nutrition formula of Claims 1 or 2 including a source of fiber.

5. The high protein liquid nutrition formula of Claims 1, 2, or 3 wherein the fat source includes 1% to 30% MCTs.

6. The high protein liquid nutrition formula of Claims 1, 2, or 3 including at least 200 mcg of molybdenum per 1,000 Kcal of formula and 140 mcg chromium.

7. The high protein liquid nutrition formula of Claims 1, 2, or 3 including at least 150% of the USRDA of Vitamin E per 1,000 Kcal of formula.

8. The high protein liquid nutrition formula of Claims 1, 2, or 3 including at least 100 mg of carnitine and taurine per 1,000 Kcal of formula.

9. The high protein liquid nutrition formula of Claims 1, 2, or 3 wherein at least 25% of the calories of the formula are protein.

**10.** The high protein liquid nutrition formula of Claims 1, 2, or 3 wherein the fat source includes a ratio of n-6 to n-3 fatty acids of 2:1 to 10:1.

**11.** The high protein liquid nutrition formula of Claim 3 wherein the source of dietary fiber includes soy polysaccharide.

**12.** The use of nutrients for the preparation of a formula for treating elevated wound healing requirements in a patient in need of same, the nutrients comprising:

a protein source;

a fat source;

a carbohydrate source;

a zinc source providing at least 150% of the USRDA of zinc per 1,000 Kcal of composition;

a Vitamin C source providing at least 500% of the USRDA of Vitamin C per 1,000 Kcal of composition;

a selenium source providing at least 100 mcg of selenium per 1,000 Kcal of composition;

a Vitamin A source providing at least 145% of the USRDA of Vitamin A per 1,000 Kcal of composition including a beta-carotene source providing at least 2.0 mg of beta-carotene per 1,000 Kcal of composition; and

a source of thiamine providing 200% of the USRDA of thiamine per 1,000 Kcal of composition.

**13.** The use according to Claim 12 wherein the elevated wound healing requirement is caused by trauma.

**14.** The use according to Claim 12 wherein the elevated wound healing requirement is caused by cancer.

**15.** The use according to Claim 12 wherein the elevated wound healing requirement is caused by burns.

**16.** The use according to Claim 12 wherein the elevated wound healing requirement is caused by pressure or vascular ulcers.

**17.** The use according to Claim 12 wherein the elevated wound healing requirement is caused by post surgical wound care.

**18.** The use according to Claim 12 wherein the elevated wound healing requirement is caused by lean body mass losses of greater than 15%.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 395 865 (NB INTERNATIONAL TECHNOLOGIES) * page 3, lines 7-27; examples 1,2; claims 1-6 * | 1-3,6,7 ,12 | A 23 L 1/305 A 23 L 1/304 A 61 K 9/00 A 61 K 9/08 |
| A | EP-A-0 259 167 (P.L. MILLMAN) * claims 1-15 * | 1-3 | |
| A | EP-A-0 511 895 (G. BARITIU) * claims 1-8 * | 1-3,14 | |
| A | PATENT ABSTRACTS OF JAPAN, vol. 10, no. 328 (C-383), 7 November 1986; & JP - A - 61135571 (ASAHI CHEM IND CO LTD) 23.06.1986 | 1 | |
| A | WO-A-8 801 861 (BAXTER TRAVENOL LABORATORIES INC.) * abstract; claims 1-5 * | 1,16 | |
| A | EP-A-0 298 179 (B. ORES et al.) * claims 1-7 * | 1,2 | TECHNICAL FIELDS SEARCHED (Int. Cl.5) A 23 L 1/00 A 61 K 9/00 |

The present search report has heen drawn up for all claims

| Place of search | Date of completion of the search | Examiner |
|---|---|---|
| BERLIN | 29-04-1993 | SCHULTZE D F |

EPO FORM 1503 03.82 (P0401)